# EUROPÄISCHE PATENTSCHRIFT

(11) **EP 0 490 383 B1**
(45) Veröffentlichungstag und Bekanntmachung des Hinweises auf die Patenterteilung: **09.10.1996**
(21) Anmeldenummer: 91121362.7
(22) Anmeldetag: 12.12.1991
(51) Int. Cl.: C07K 14/16, C07K 7/08, A61K 38/04, A61K 38/16

(54) **Peptide des HIV-gag Proteins, ihre Herstellung und Verwendung**
Peptides of the HIV-gag protein, their preparation and use
Peptides de la protéine gag d'HIV, leur procédé de préparation et leur utilisation

(30) Priorität: 14.12.1990 DE 4039925
(43) Veröffentlichungstag der Anmeldung: 17.06.1992
(60) Teilanmeldung: 96100385.2
(73) Patentinhaber: BEHRINGWERKE Aktiengesellschaft, 35001 Marburg (DE)
(72) Erfinder: Niedrig, Matthias, Dr., W-3550 Marburg (DE); Modrow, Susanne, Dr., W-8000 München 71 (DE); Wolf, Hans, Prof. Dr., W-8130 Starnberg (DE)

(56) Entgegenhaltungen:
- WO-A-86/06414

## Beschreibung

Die Erfindung betrifft ausgewählte Peptide der HIV-gag-Sequenz, die eine Inhibition der Virussynthese bewirken. Bei Testung von 41 sequenziellen Peptiden auf Hemmung der HIV-Vermehrung wurde gefunden, daß die überlappenden Peptide 4 und 5 mit der Aminosäuresequenz WASRELERFAVNPGLLETSEGCRQ und NPGLLETSEGCRQILGQLQPSLQT aus dem p17 Submembranprotein und die beiden Peptide 28 und 29 mit den Aminosäuresequenzen ANPDCKTILKALGPAATLEEMMTA und AATLEEMMTACQGVGGPGHKA aus dem p24 Coreprotein die Vermehrung von HIV hemmen (überlappende Bereiche sind jeweils unterstrichen). Solche oder ähnliche Peptide, die mindestens die überlappenden Bereiche enthalten, sind als Arzneimittel zur Bekämpfung von HIV-Infektionen geeignet.

Die durch das HIV verursachte Erkrankung AIDS stellt eine große Anforderung an die wissenschaftliche Forschung bei der Entwicklung therapeutisch wirksamer Substanzen und neuer Vakzinen dar. Auch wenn sich die Forschung über das gesamte Spektrum möglicher Therapieansätze erstreckt, versprechen doch nur sehr wenige Substanzen Aussicht auf eine neue erfolgreiche Therapie. Bisher ist als einzige zugelassene anti-HIV wirksame Substanz ^{R}Retrovir mit dem Wirkstoff Zidovudin der Firma Wellcome auf dem Markt. Dieses Nukleotidanalog, Azidothymidin (AZT) hemmt sehr wirkungsvoll in vitro und in vivo die HIV-spezifische Reverse Transkriptase, ist jedoch nicht frei von nachteiligen Eigenschaften. Bisher bestehen keine Alternativen zu der AZT-Therapie.

Untersuchungen über die HIV-Virusstruktur und die Rolle der verschiedenen Virusstrukturproteine bei der Virusreifung führten zu der Erkenntnis, daß sich hier mehrere Ansätze für eine wirkungsvolle Inhibition der Virussynthese bieten, wobei die Inhibition der viralen Protease durch Chemotherapeutika nur ein Beispiel darstellt, das zur Zeit von verschiedenen Arbeitsgruppen bearbeitet wird. Die Tatsache, daß die gag-Sequenz zu den hochkonservierten Regionen im HIV-Genom zählt, läßt vermuten, daß es sich hierbei um ein für die Virusvermehrung sehr wichtiges Protein handelt; die geringen Sequenzunterschiede bei verschiedenen HIV-Isolaten unterstützen diese Vermutung (siehe Tab. 1). Der Vorgang der Virussynthese wird eingeleitet durch die gag-Proteinsynthese und die notwendige Myristoylierung des gag-Proteins. Der weitere Zusammenbau findet an der Lipidmembran der infizierten Zelle statt. Durch Aneinanderlagern von gag-Proteinen kommt es zur Ausstülpung der Zellmembran und zur Abschnürung ("Budding") von "Teilchen" bzw. unreifen Viren. Daß dieser Vorgang auch abläuft, wenn nur das gag-Gen in die Zelle eingeschleust wird, konnte durch Untersuchungen mit rekombinanten gag-Sequenzen in Vaccinia-bzw. Baculovectoren gezeigt werden (Karacostas et al. (1989) Human immunodeficiency virus-like particles produced by vaccinia virus expression vector. Proc. Natl. Acad. Sci. USA, Vol. 86, 8965-8967; Gheysen et al. (1989) Assembly and release of HIV-1 precursor prgag55 virus-like particles from recombinant baculovirus-infected insect cells. Cell, 59, 103-112). Andere Publikationen belegen, daß es innerhalb der gag-Sequenz für das "Processing" wichtige Bereiche gibt. Durch Einführung gezielter Mutationen innerhalb der gag-Sequenz, wurden mehrere für das "Processing" relevante Bereiche identifiziert (Göttlinger et al. (1990) Role of capsid precursor processing and myristoylation in morphogenesis and infectivity of human immunodeficiency virus type 1. Proc. Natl. Acad. Sci. USA, Vol. 86, 5781-5785).

Die internationale Patentanmeldung WO 86/06414 beschreibt Peptide mit einer Länge von mindestens 5 Aminosäuren, die in spezifischen Bindungstests für die Messung von Antikörper gegen das LAV/HTLV-III Virus und für die Messung von LAV/HTLV-III Antigen oder as Immunogen Verwendung finden können.

Allerdings gab es keinerlei Hinweise darauf, daß bestimmte Bereiche der gag Sequenz für Peptide kodieren, die die Virussynthese von HIV hemmen.

In den der Erfindung zugrunde liegenden Versuchen wurden 41 einander überlappende synthetische Peptide der HIV-1 gag Sequenz auf ihre die Virussynthese hemmende Funktion in einem in vitro Test untersucht. Die 24 Aminosäuren langen Peptide wurden analog der von Ratner et al. (Complete nucleotide sequence of the AIDS virus, HTLV-III, Nature, 313, 277-284 (1985)) publizierten Sequenz synthetisiert.

Diese Peptide wurden in unterschiedlichen Konzentrationen (200-40 µg/ml) auf frisch infizierte Jurkat Zellen gegeben Die Infektion erfolgte mit 100-1000 TCID₅₀. Die Analyse der Infektion erfolgte durch mikroskopische Beurteilung und Untersuchung des Überstandes auf Gehalt an infektiösem HIV. Hierfür wurde Zellüberstand auf nicht infizierte Jurkatzellen gegeben. Nach zwei Wochen Inkubation wurde diese Nachweiszellkultur mikroskopisch und durch Reverse Transkriptase Assay auf HIV untersucht. Hierbei wurde gefunden, daß es zwei gag-Bereiche gibt, die einen hemmenden Effekt auf die Virussynthese ausüben. Diese beiden Bereiche sind jeweils durch zwei Peptide repräsentiert, der erste (Peptid 4+5) liegt innerhalb des p17, der zweite (Peptid 28+29) innerhalb des p24.

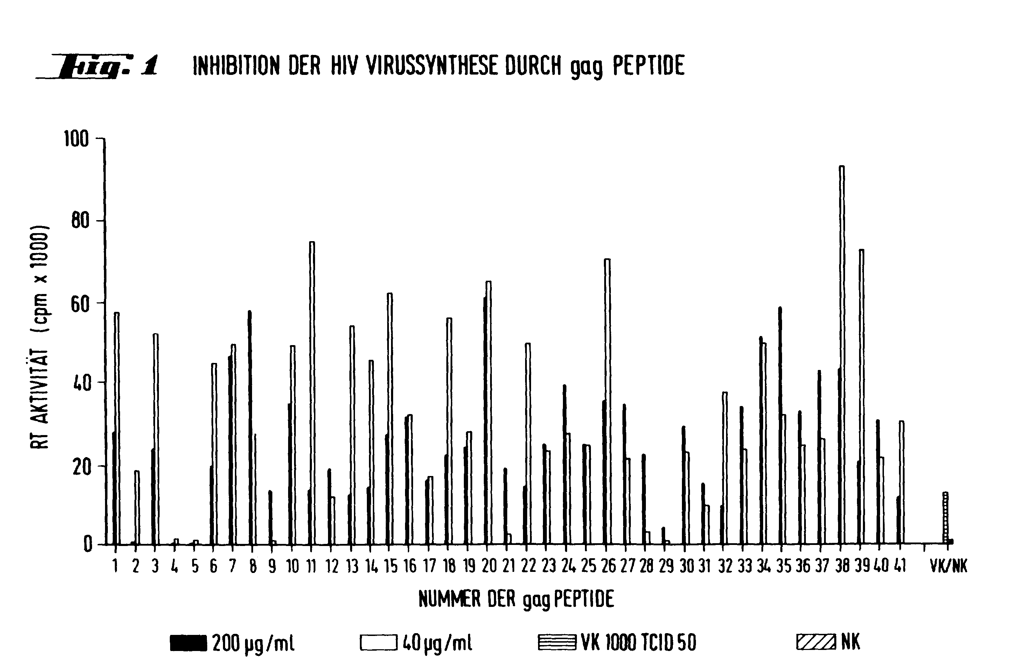

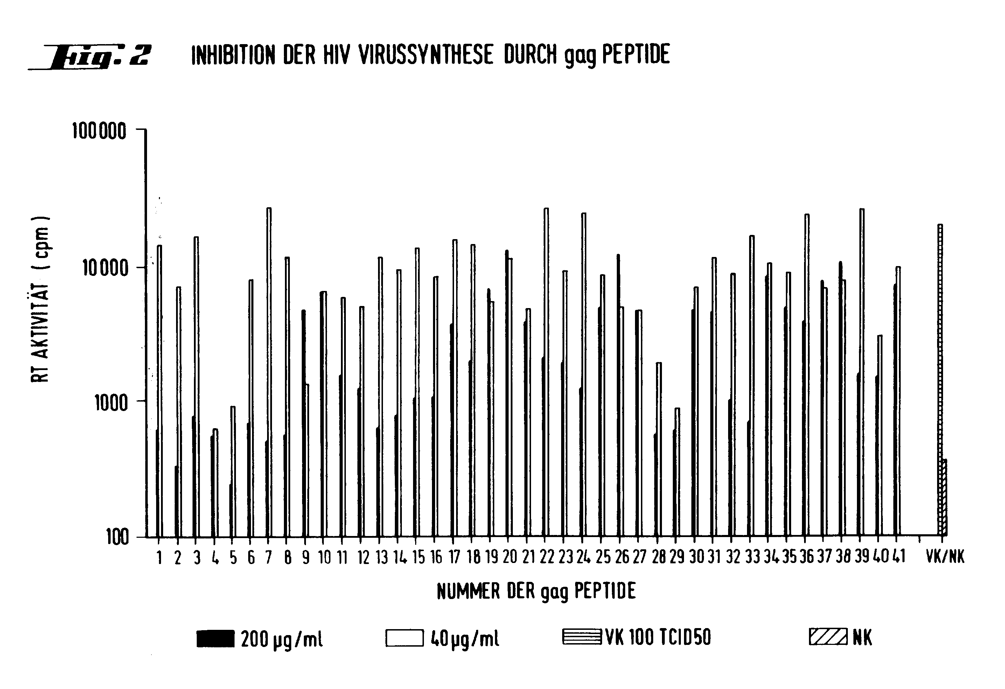

Wie aus Tab. 4 ersichtlich, läßt sich in HIV-infizierten Zellen nach Behandlung mit diesen Peptiden kein p24 nachweisen. Der überstand dieser Zellkulturen enthält kein infektiöses HIV, das zur Infektion einer Kontrollkultur in der Lage wäre. Die Figuren 1 und 2 geben die Reverse Transkriptase (RT) Aktivität der beiden Nachweiszellkulturen wieder. Die vier ausgewählten Peptide vermögen die HIV-Synthese in Konzentrationen von 200-40 µg/ml sehr wirkungsvoll zu inhibieren. (Einzelheiten zum Nachweis der HIV-Inhibition siehe Beispiele).

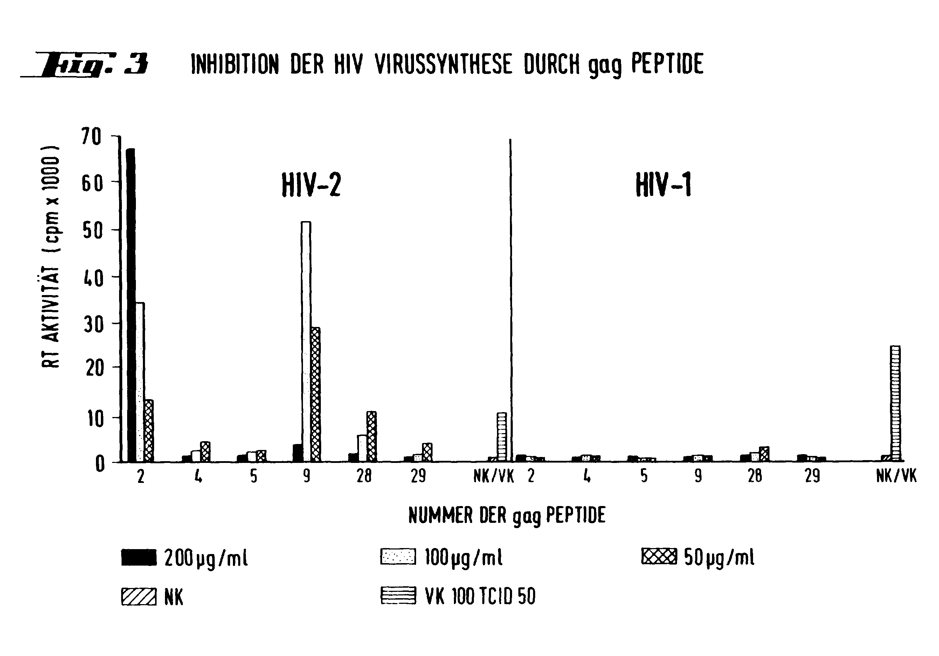

Der Einfluß von unterschiedlichen Konzentrationen an infektiösem Virus auf den Versuchsablauf ist relativ gering und spiegelt sich hauptsächlich in der Höhe der gemessenen RT-Aktivität wieder (vgl. Versuch 1, Fig. 1; Versuch 2, Fig. 2). Auch die unterschiedlichen Konzentrationen an zugesetzten Peptiden haben keinen signifikanten Einfluß auf die Inhibition, was vermuten läßt, daß die Konzentration der zugegebenen Peptide noch weiter abgesenkt werden kann, ohne die Wirkung zu beeinflussen. Das Ergebnis des dritten Versuches ist in Fig. 3 wiedergegeben. Die hier dargestellten Reverse Transkriptase Aktivitäten sind nach zwei Wochen Inkubation der Nachweiszellkulturen aus dem Zellkulturüberstand nach fünffacher Konzentration gemessen. Auch hier zeigt sich der starke inhibierende Einfluß der Peptide 2, 4, 5, 9, 28, 29 auf die HIV-1 Synthese. Die Inhibition der HIV-2 Synthese durch die Peptide 4, 5, 28, 29 ist auch sehr deutlich zu erkennen. Dennoch scheinen hier die zugegebenen Peptide schwächer in ihrer inhibierenden Wirkung zu sein. So kommt es bei den Peptiden 2 und 9 zu keiner Inhibition, bei 28 ist nur die 200 µg/ml Dosis zu einer vollständigen Hemmung der Virussynthese in der Lage. Bei allen 41 untersuchten Petiden konnte nur für das Peptid Nr. 9 eine Zelltoxizität festgestellt werden.

Die Erfindung betrifft folglich Peptide, die die Peptidsequenz NPGLLETSEGCRQ enthält und nicht größer als 50 Aminosäuren, insbesondere nicht größer als 40 Aminosäuren, vor allem nicht größer als 30 Aminosäuren, vorzugsweise nicht größer als 20 Aminosäuren sind. Insbesondere bevorzugt sind Peptide, die die Peptide 4, 5, 28 oder 29, enthalten.

Eine weitere Ausführungsform der Erfindung sind Peptide, die die Peptidsequenz NPGLLETSEGCRQ enthalten, wobei die Sequenzen am N- und/oder am C-Terminus um bis zu 7 Aminosäuren, vorzugsweise um bis zu 4 Aminosäuren verkürzt werden können, ohne jedoch eine Länge von 6 Aminosäuren zu unterschreiten. Häufig ist es auch vorteilhaft, das erfindungsgemäße Peptid um eine oder mehrere Aminosäuren, beispielsweise Cystein, zu verlängern, um das Verknüpfen der Peptide untereinander oder an einen Träger zu erreichen.

Des weiteren umfaßt die Erfindung Derivate der Peptidsequenz NPGLLETSEGCRQ, bei denen eine oder mehrere der folgenden Substitutionen nach dem Fachmann bekannten Methoden durchgeführt werden können: Asparagin gegen Glutamin, bzw. Glutamin gegen Asparagin, Prolin gegen Hydroxyprolin, Leucin gegen Isoleucin oder Norleucin, Glutaminsäure gegen Asparaginsäure, Threonin gegen Serin bzw. Serin gegen Threonin, Cystein gegen Serin, Arginin gegen Lysin, Alanin gegen Glycin und/oder Methionin gegen Norleucin. Bevorzugt ist der Ersatz einer natürlichen Aminosäure durch eine nicht natürliche Aminosäure, wie beispielsweise Hydroxyprolin oder Norleucin.

Dadurch kann verhindert werden, daß körpereigene Proteasen das Derivat spalten, was im allgemeinen zu einer Verlängerung der Halbwertszeit führt. Auch können die Derivate eine verbesserte Löslichkeit und/oder eine bessere Resorption besitzen im Vergleich zu der erfindungsgemäßen Peptidsequenz.

Weitere Gegenstände der Erfindung sind die proteinchemische oder gentechnische Herstellung sowie die Verwendung des erfindungsgemäßen Peptids als Arzneimittel.

Vorzugsweise wird das erfindungsgemäße Peptid proteinchemisch hergestellt, beispielsweise nach BARANI, G. und MERRIFIELD, R.B. in "The Petides, Analysis, Synthesis and Biology", Vol. 2, Academic Press 1980, Ed. Erhard Gross, Johannes Meyerhofer.

Die Erfindung ist in den Beispielen näher erläutert und in den Patentansprüchen enthalten.

### Beispiel 1: Allgemeine Grundlagen

**Zellen**. Es wurden permanent wachsende T-Lymphozyten (Jurkat- oder H9-Zellen) für die in vitro-Experimente verwendet. Als Medium wurde konventionelles RPMI 1640 mit 10% FKS, 2% NaHCO₃ (5%ig), 1% Penicillin/Streptavidinlösung und 2 mg/l Polybrene verwendet. Die Experimente wurden wahlweise in 96 Loch-Mikrotiterplatten (Nunc) oder 24 Loch-Platten (Nunc) durchgeführt.

**Viren.** Es wurde HIV-1 virus Stamm HTLV-IIIB und HIV-2 Stamm ROD verwendet.

**Peptidsynthese und Peptidreinigung.** Die Peptide wurden nach der von Ratner et al. (1985) publizierten HIV-1 Sequenz auf einem Syntheseautomaten (Milligen 9050,

Milligen GmbH, Eschborn, FRG) unter Einsatz von Fmocgeschützten Aminosäuren (Bachem AG, Heidelberg, FRG) hergestellt (Atherton et al. (1978): A mild procedure for solid phase peptide synthesis; Use of Fluorenylmethyloxycarbonyl amino acids: J. Chem. Soc. Chem. Commun. 13, 539-540). Als Trägermaterial wurde jeweils ^{R}Tentagelharz mit säurestabilem AM-Linker verwendet (Rapp-Polymere, Tübingen, FRG). Die Aminosäuren wurden jeweils vor der Kopplung in DMF gelöst und in Hydroxybenzotriazol-aktivierte Ester überführt. Zur Kopplung wurden Schnellsynthesezyklen mit 10-minütiger Raktionszeit eingesetzt. Die Fmoc-Gruppe wurde in Anschluß mit 20% Piperidin abgespalten. Die Vollständigkeit dieser Reaktion wurde fluorimetrisch überprüft.

Nach vollendeter Synthese wurde das Harz mit dem geschützten Peptid in 50% TFA/DCM suspendiert. Als Scavenger wurden 1% Anisol, 1% m-Kresol, 1% Phenol und, falls Trp in der jeweiligen Sequenz enthalten war, 5% Mercaptoethanol zugesetzt. Die Bindung zum Harz und die Trt- und tBoc-Schutzgruppen wurden in 4-stündiger Inkubationszeit bei Raumtemperatur unter Argon-Schutzgas abgespalten. Zur Entfernung der Mtr-Schutzgruppe des Arg wurde das abgespaltene Peptid vom Harz abgetrennt, das Lösungsmittel im Rotationsverdampfer abgezogen, die verbleibende Festsubstanz in 100% incl. den übliche Scavenger (siehe oben) über Nacht inkubiert. Die entschützten Peptide wurden nach Abzug des Lösungsmittel in 50% Essigsäure gelöst, in einem großen Volumen eiskalter t-Butyl-ethyl-ether gefällt, mehrmals gewaschen und lyophilisiert. Die getrocknete Rohsubstanz wurde in 1,5% Ammoniumbicarbonat aufgenommen. Unlösliche Bestandteile wurden abfiltriert. Das Filtrat wurde erneut getrocknet. Die Reinigung der Peptide erfolgte auf einer halbpräperativen Propep Reversed Phase HPLC-Säule (C₂/C₁₈ Copolymer, Pharmacia/LKB, Freiburg, FRG), wobei zur Elution üblicherweise Gradienten von 0-70% Acetonitril in 0,1% TFA unter Heliumbegasung eingesetzt wurden. Die Sequenzen der gereinigten Peptide wurden auf einem Gasphasensequenzer (Applied Biosystems, Westerstadt, FRG) überprüft. Die eingesetzten Peptide entsprechen den in Tab. 2 abgebildeten Aminosäuresequenzen. Es ist selbstverständlich möglich, die o.g. Peptide auch gentechnisch z.B. als geeignete Fusionsproteine in pro- oder eukaryontischen Zellsystemen herzustellen.

**Reverse Transkriptase Assay.** Der Mikrotest zum Nachweis der viralen Reversen Transkriptase wurde nach Gregersen et al. durchgeführt (Gregersen et al. (1988) Detection of human immunodeficiency virus and other retroviruses in cell culture supernatants by a reverse transcriptase microassay. J. Virol. Methods 19, 161-168). Zellkulturüberstände wurden durch PEG-Fällung fünffach ankonzentriert. Als positive Kontrolle (VK) dienten Überstände unbehandelter infizierter Zellkulturen, als negative Kontrolle (NK) dienten Zellkulturüberstände nicht infizierter Zellen. Der bei der NK gemessene Wert wurde verdoppelt und diente als Ausschlußwert für negative Zellen. Zur besseren Übersichtlichkeit sind die gemessenen RT-Werte in der logarithmischen Darstellung wiedergegeben (siehe auch Fig. 1, 2, 3).

### Beispiel 2: Nachweis der Inhibition der HIV-Synthese

Der Versuchsaufbau ist schematisch in der Tab. 3 wiedergegeben. Es wurden jeweils zwei Versuche zur Inhibition der HIV-Synthese mit allen zur Verfügung stehenden 41 Peptiden durchgeführt.

Bei dem ersten Versuch wurden jeweils 50 µl 1x10⁶ Jurkat-Zellen/ml in die Vertiefung von 96 Loch-Platten pipettiert und mit jeweils 50 µl (1000 TCID₅₀) HTLV-IIIB infiziert. Die gag-Peptid-Konzentration wurde auf 200 µg/ml bzw. 40 µg/ml eingestellt. Nach einer Woche wurden 100 µl überstand abgenommen und eventuell noch vorhandene Zellen durch Zentrifugation abgetrennt. Anschließend wurden 80 µl überstand auf eine nicht infizierte Jurkat-Zellkultur gegeben. Der Nachweis von Virusantigen in den infizierten Zellen erfolgte mittels Immunmarkierung nach Western Blot. Hierfür wurden die nach dem Inhibitionsversuch verbleibenden Zellen in 2-fach SDS-PAGE Probenpuffer aufgenommen und in einem 14%igem PAG aufgetrennt. Danach wurden die aufgetrennten Proteine auf eine Nitrocellulosemembran geblottet und zum Nachweis viraler Proteine mit einem anti-p24 HIV-1 monoklonalen Antikörper inkubiert. Die spezifische Anfärbung erfolgte über einen zweiten anti-Maus Antikörper mit gekoppelter alkalischer Phosphatase. Die Analyse von infektiösem HIV im überstand der Peptid behandelten Zellen erfolgte mittels Reverse Transkriptase Test.

Der zweite Versuch wurde analog dem ersten Versuchsansatz mit einer geringeren Konzentration an infektiösen Einheiten (100 TCID₅₀) durchgeführt. Die Analysen auf infektiöses Virus im Zellkulturüberstand bzw. HIV-Protein in den infizierten Zellkulturen wurden analog dem ersten Versuch durchgeführt. In einem dritten Versuchsansatz wurden ausgewählte Peptide (2, 4, 5, 9, 28, 29), die einen inhibierenden Einfluß auf die HIV-1 Synthese ausübten, unter gleichen Bedingungen auf ihre inhibierende Wirkung bei HIV-2 getestet. Für diesen Versuch wurden H9-Zellen mit 100 TCID₅₀ HIV-2 infiziert. Die Analyse der Infektion erfolgte analog der zwei vorhergehenden Experimente.

In den ersten beiden Experimenten wurden zwei Bereiche innerhalb der gag-Sequenz identifiziert, die jeweils durch zwei überlappende Peptide repräsentiert sind, welche einen inhibierenden Einfluß auf die HIV-Synthese ausüben. Die Peptide 4 und 5 liegen innerhalb der p17 Proteinsequenz, während 28, 29 innerhalb der p24 Proteinsequenz liegen.

In Tab. 1 sind bei dem Vergleich verschiedener HIV-gag-Proteinsequenzen die durch die Peptide 4, 5 und 28, 29 dargestellten Bereiche durch Kästchen markiert. Tab. 2 listet die Aminosäuresequenz der 41 einander überlappenden Sequenzen auf.

### Legenden:

**Tab. 1:** Vergleich verschiedener HIV-gag-Proteinsequenzen. Die durch die Peptide 4, 5 und 28, 29 dargestellten Bereiche sind durch Kästen markiert.

**Tab. 2:** Aminosäuresequenz der 41 einander überlappenden synthetischen gag-Peptide. Die Peptide wurden analog der von Ratner et al. publizierten Sequenz synthetisiert.

**Tab. 3:** Schematischer Versuchsaufbau der durchgeführten und geplanten Versuche zum Nachweis der Inhibition der HIV-Synthese durch gag-Peptide.

**Tab. 4:** Inhibition der HIV-Synthese mit gag-Peptiden. Auswertung des p24-Nachweises in HIV-infizierten Zellen nach Peptidbehandlung zwei Wochen nach Infektion. Zellen der Ausgangsplatte wurden mit 2fachen Probenpuffer versetzt und im 14%-igen PAG aufgetrennt. Nach dem Blotten auf Nitrocellulose erfolgte der Nachweis von HIV-Protein durch spezifische anti-p24 MAbs. - = keine Reaktion nachweisbar, (+) = schwache Reaktion nachweisbar, + = gute positive Reaktion nachweisbar, ++ = starke Reaktion nachweisbar.

**Fig. 1:** Inhibition der HIV Virussynthese durch gag Peptide (Versuch 1 ). Es wurden 1000 TCID₅₀ HIV-1 für die Infektion der Jurkat-Zellen eingesetzt. Reverse Transkriptase Aktivität gemessen in cpm im Zellkulturüberstand der Nachweiszellkultur. Der Überstand wurde durch PEG-Fällung fünffach ankonzentriert. VK = Viruskontrolle; NK = Negativ-Kontrolle. Der doppelte NK-Wert diente als Ausschluß für negative Zellkulturen. Der besseren Übersichtlichkeit wegen sind die RT-Werte in der Abbildung in der logarithmischen Darstellung wiedergegeben.

**Fig. 2** Inhibition der HIV-Synthese durch gag Peptide (Versuch 2). Es wurden 100 TCID₅₀ HIV-1 für die Infektion der Jurkat-Zellen eingesetzt. Reverse Transkriptase Aktivität gemessen in cpm im Zellkulturüberstand der Nachweiszellkultur. Der Überstand wurde durch PEG-Fällung fünffach ankonzentriert. VK = Viruskontrolle; NK = Negativ Kontrolle. Der doppelte NK-Wert diente als Ausschluß für negative Zellkulturen. Der besseren Übersichtlichkeit wegen sind die RT-Werte in der Abbildung in der logarithmischen Darstellung wiedergegeben.

**Fig. 3** Inhibition der HIV-Synthese durch gag Peptide (Versuch 3). Es wurden 100 TCID₅₀ HIV-1 bzw. HIV-2 für die Infektion der Jurkat bzw. H9-Zellen eingesetzt. Reverse Transkriptase Aktivität gemessen in cpm im Zellkulturüberstand der Nachweiszellkultur. Der Überstand wurde durch PEG-Fällung fünffach ankonzentriert. VK = Viruskontrolle; NK = Negativ Kontrolle. Der doppelte NK-Wert diente als Ausschluß für negative Zellkulturen. Der besseren Übersichtlichkeit wegen sind die RT-Werte in der Abbildung in der logarithmischen Darstellung wiedergegeben.

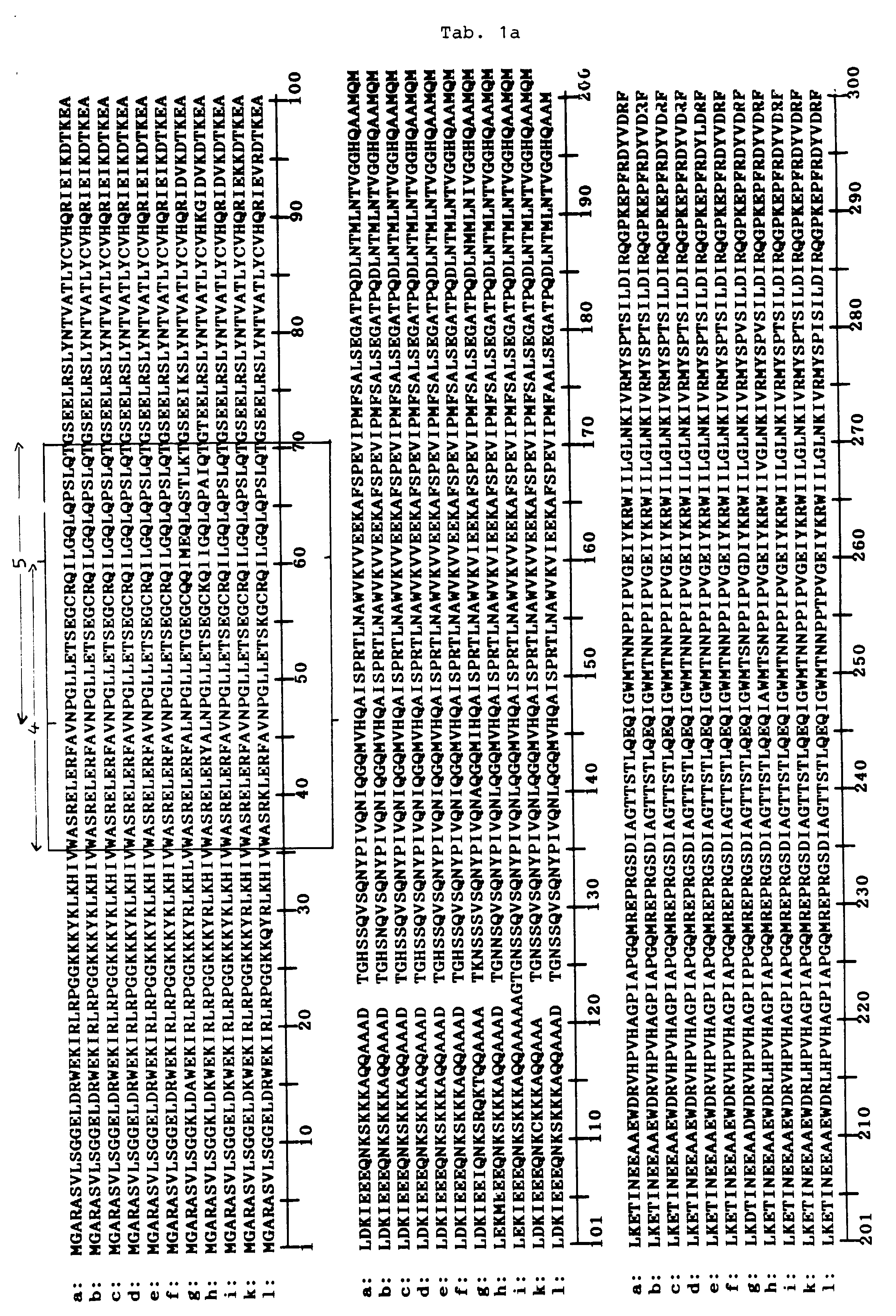

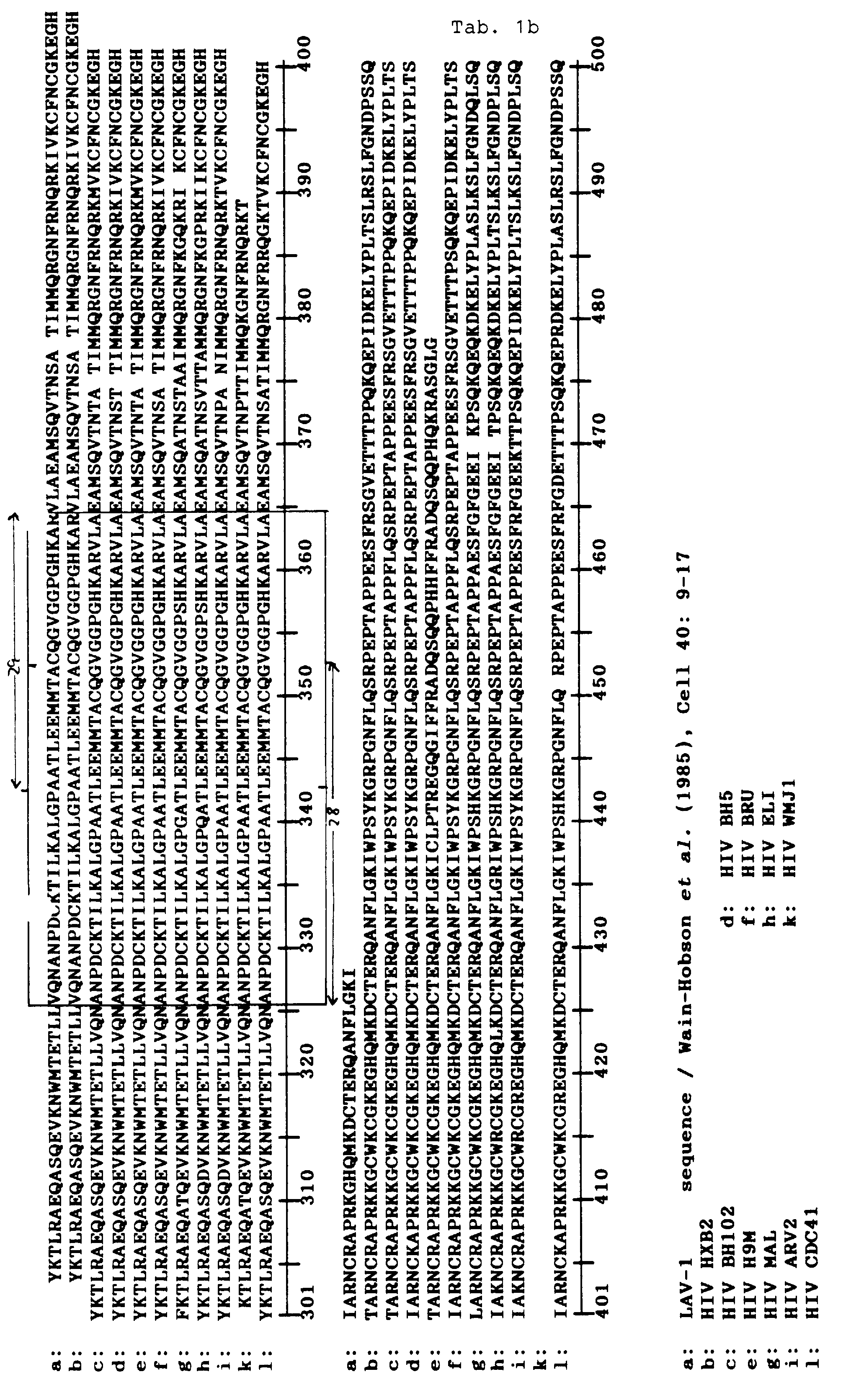

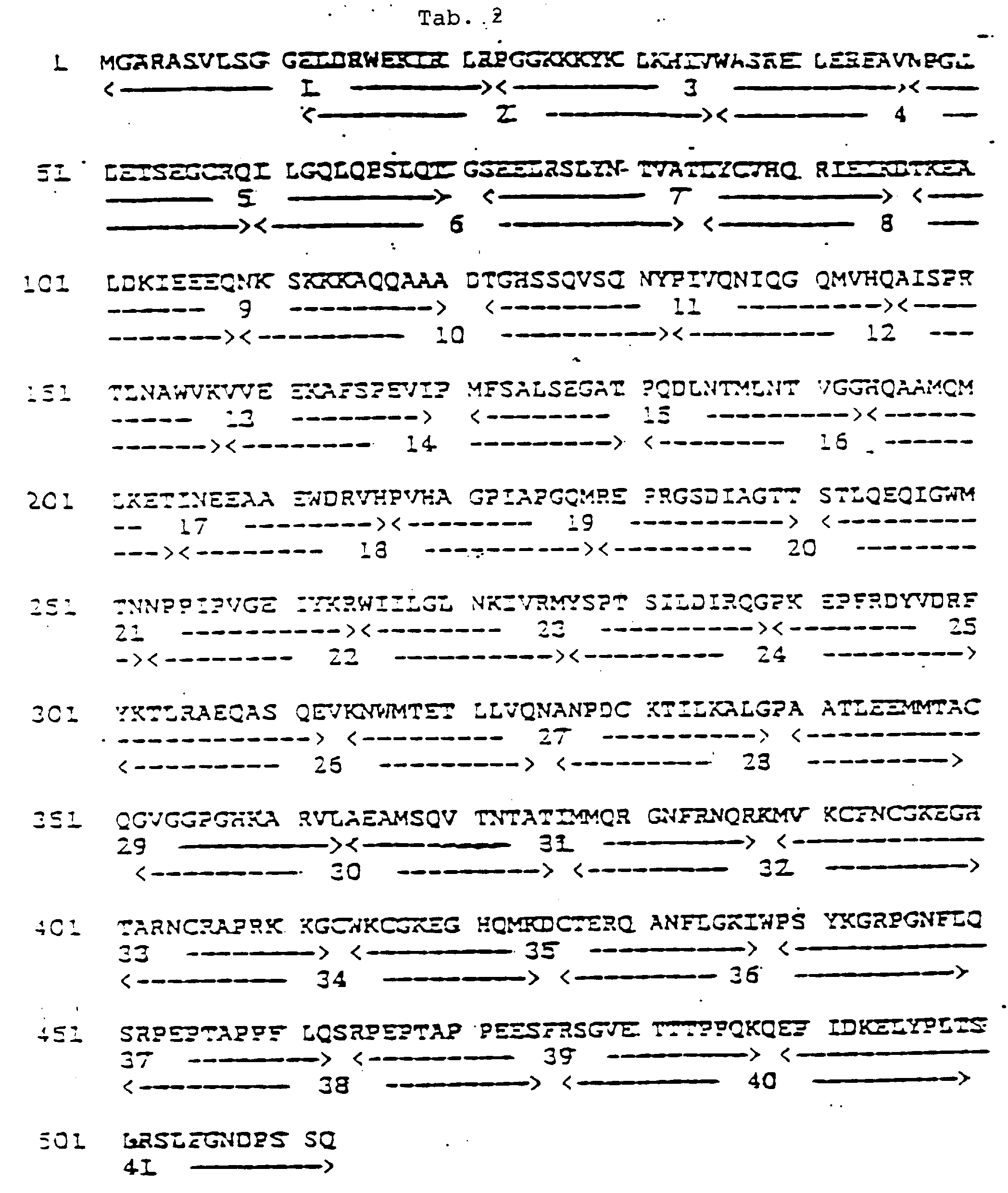

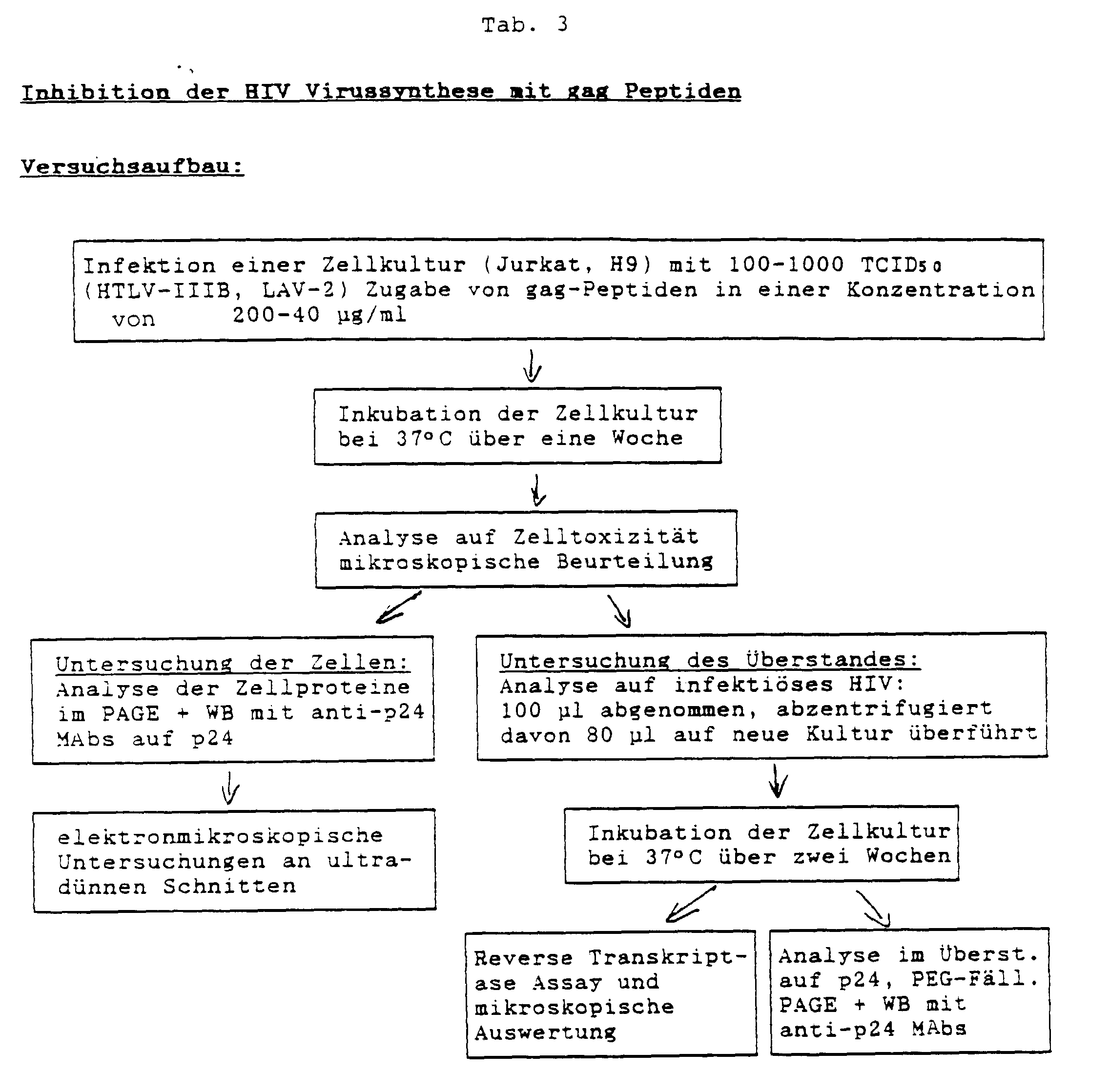

**Tab.4**

| Inhibtion der HIV-Virussynthese mit gag-Peptiden: | |
|---|---|
| Peptid Nr.: | p24 Nachweis: |
| 1 | + |
| 2 | + |
| 3 | + |
| 4 | - |
| 5 | (+) |
| 6 | ++ |
| 7 | ++ |
| 8 | ++ |
| 9 | + |
| 10 | + |
| 11 | + |
| 12 | + |
| 13 | + |
| 14 | + |
| 15 | + |
| 16 | + |
| 17 | + |
| 18 | ++ |
| 19 | + |
| 20 | + |
| 21 | + |
| 22 | + |
| 23 | + |
| 24 | + |
| 25 | ++ |
| 26 | ++ |
| 27 | ++ |
| 28 | (+) |
| 29 | - |
| 30 | ++ |
| 31 | + |
| 32 | + |
| 33 | + |
| 34 | ++ |
| 35 | ++ |
| 36 | ++ |
| 37 | + |
| 38 | + |
| 39 | + |
| 40 | + |
| 41 | + |

## Patentansprüche (Patentansprüche für folgende(n) Vertragsstaat(en): AT, BE, CH, DE, DK, FR, GB, IT, LI, LU, NL, SE)

1. Peptide des HIV-gag Proteins, die die Peptidsequenz NPGLLETSEGCRQ enthält und nicht größer als 50 Aminosäuren sind.

2. Peptide nach Anspruch 1, die die Peptide P4 oder P5 enthalten.

3. Peptide nach Anspruch 1 oder 2, dadurch gekennzeichnet, daß die Peptidsequenz NPGLLETSEGCRQ am N- und/oder am C-Terminus um bis zu 7 Aminosäuren verkürzt ist, jedoch jeweils nicht kürzer als 6 Aminosäuren ist.

4. Peptide nach mindestens einem der Ansprüche 1 bis 3, die eine oder mehrere der folgenden Aminosäureaustausche besitzen: Asparagin gegen Glutamin, bzw. Glutamin gegen Asparagin, Prolin gegen Hydroxyprolin, Leucin gegen Isoleucin oder Norleucin, Glutaminsäure gegen Asparaginsäure, Threonin gegen Serin bzw. Serin gegen Threonin, Cystein gegen Serin, Arginin gegen Lysin, Alanin gegen Glycin und/oder Methionin gegen Norleucin.

5. Peptide nach mindestens einem der Ansprüche 1 bis 4 als Arzneimittel.

6. Therapeutische Zubereitung, enthaltend mindestens ein Peptid nach mindestens einem der Ansprüche 1 bis 5 zusammen mit inertem Trägermaterial.

7. Verfahren zur Herstellung der Peptide nach mindestens einem der Ansprüche 1 bis 4, dadurch gekennzeichnet, daß das Verfahren ein proteinchemisches Verfahren ist.

8. Verwendung von Peptiden nach Anspruch 1 bis Anspruch 5 zur Herstellung eines Arzneimittels zur Bekämpfung von AIDS.

## Patentansprüche (Patentansprüche für folgende(n) Vertragsstaat(en): GR, ES)

1. Verfahren zur Herstellung von Peptiden des HIV-gag Proteins, dadurch gekennzeichnet, daß die Peptide die Peptidsequenz NPGLLETSEGCRQ enthalten und nicht größer als 50 Aminosäuren sind.

2. Verfahren nach Anspruch 1, dadurch gekennzeichnet, daß die Peptide P4 oder P5 enthalten.

3. Verfahren nach Anspruch 1 oder 2, dadurch gekennzeichnet, daß die Peptidsequenz NPGLLETSEGCRQ am N- und/oder am C-Terminus um bis zu 7 Aminosäuren verkürzt ist, jedoch jeweils nicht kürzer als 6 Aminosäuren ist.

4. Verfahren nach mindestens einem der Ansprüche 1 bis 3, dadurch gekennzeichnet, daß die Peptidsequenz NPGLLETSEGCRQ eine oder mehrere der folgenden Aminosäureaustausche besitzt: Asparagin gegen Glutamin, bzw. Glutamin gegen Asparagin, Prolin gegen Hydroxyprolin, Leucin gegen Isoleucin oder Norleucin, Glutaminsäure gegen Asparaginsäure, Threonin gegen Serin bzw. Serin gegen Threonin, Cystein gegen Serin, Arginin gegen Lysin, Alanin gegen Glycin und/oder Methionin gegen Norleucin.

5. Verfahren zur Herstellung eines Arzneimittels, dadurch gekennzeichnet, daß Peptide, hergestellt nach mindestens einem der Ansprüche 1 bis 4, verwendet werden.

6. Verfahren zur Herstellung eines Arzneimittels nach Anspruch 5, dadurch gekennzeichnet, daß zusätzlich ein inertes Trägermaterial verwendet wird.

7. Verfahren zur Herstellung eines Arzneimittels nach Anspruch 5 oder 6, dadurch gekennzeichnet, daß das Arzneimittel zur Bekämpfung von AIDS verwendet wird.

## Claims (Claims for the following Contracting State(s): AT, BE, CH, DE, DK, FR, GB, IT, LI, LU, NL, SE)

1. A peptide of the HIV gag protein which contains the peptide sequence NPGLLETSEGCRQ and is not larger than 50 amino acids.

2. A peptide as claimed in claim 1, which contains the peptide P4 or P5.

3. A peptide as claimed in claim 1 or 2, wherein the peptide sequence NPGLLETSEGCRQ is truncated by up to 7 amino acids at the N and/or at the C terminus but is not shorter than 6 amino acids in each case.

4. A peptide as claimed in at least one of claims 1 to 3, which has one or more of the following amino-acid exchanges: asparagine by glutamine, or glutamine by asparagine, proline by hydroxyproline, leucine by isoleucine or norleucine, glutamic acid by aspartic acid, threonine by serine or serine by threonine, cysteine by serine, arginine by lysine, alanine by glycine and/or methionine by norleucine.

5. A peptide as claimed in at least one of claims 1 to 4 as pharmaceutical.

6. A therapeutic composition containing at least one peptide as claimed in at least one of claims 1 to 5, together with an inert vehicle.

7. A process for the preparation of a peptide as claimed in at least one of claims 1 to 4, wherein the process is a process of protein chemistry.

8. The use of a peptide as claimed in claim 1 to claim 5 for the preparation of a pharmaceutical for controlling AIDS.

## Claims (Claims for the following Contracting State(s): GR, ES)

1. A process for the preparation of peptides of the HIV gag protein, wherein the peptides contain the peptide sequence NPGLLETSEGCRQ and are not larger than 50 amino acids.

2. The process as claimed in claim 1, wherein the peptides contain P4 or P5.

3. The process as claimed in claim 1 or 2, wherein the peptide sequence NPGLLETSEGCRQ is truncated by up to 7 amino acids at the N and/or at the C terminus but is not shorter than 6 amino acids in each case.

4. The process as claimed in at least one of claims 1 to 3, wherein the peptide sequence NPGLLETSEGCRQ has one or more of the following amino-acid exchanges: asparagine by glutamine, or glutamine by asparagine, proline by hydroxyproline, leucine by isoleucine or norleucine, glutamic acid by aspartic acid, threonine by serine or serine by threonine, cysteine by serine, arginine by lysine, alanine by glycine and/or methionine by norleucine.

5. A process for the preparation of a pharmaceutical, which comprises using peptides prepared as claimed in at least one of claims 1 to 4.

6. The process for the preparation of a pharmaceutical as claimed in claim 5, wherein an inert vehicle is additionally used.

7. The process for the preparation of a pharmaceutical as claimed in claim 5 or 6, wherein the pharmaceutical is used for controlling AIDS.

## Revendications (Revendications pour l'(les) Etat(s) contractant(s) suivant(s): AT, BE, CH, DE, DK, FR, GB, IT, LI, LU, NL, SE)

1. Peptides de la protéine gag du VIH, qui contiennent la séquence peptidique NPGLLETSEGCRQ et ne comportent pas plus de 50 aminoacides.

2. Peptides selon la revendication 1, contenant le peptide P4 ou P5.

3. Peptides selon la revendication 1 ou 2, caractérisés en ce que la séquence peptidique NPGLLETSEGCRQ est raccourcie de jusqu'à 7 aminoacides à l'extrémité N-terminale et/ou à l'extrémité C-terminale, mais dans chaque cas n'est pas plus courte que 6 aminoacides.

4. Peptides selon au moins l'une des revendications 1 à 3, comportant un ou plusieurs des remplacements d'aminoacides suivants: l'asparagine par la glutamine ou la glutamine par l'asparagine, la proline par l'hydroxyproline, la leucine par l'isoleucine ou la norleucine, l'acide glutamique par l'acide aspartique, la thréonine par la sérine ou la sérine par la thréonine, la cystéine par la sérine, l'arginine par la lysine, l'alanine par la glycine et/ou la méthionine par la norleucine.

5. Peptides selon au moins l'une des revendications 1 à 4, en tant que médicament.

6. Composition thérapeutique contenant au moins un peptide selon au moins l'une des revendications 1 à 5, conjointement avec un véhicule.

7. Procédé pour la préparation des peptides selon au moins l'une des revendications 1 à 4, caractérisé en ce que le procédé est un procédé de la chimie des protéines.

8. Utilisation des peptides selon les revendications 1 à 5, pour la fabrication d'un médicament destiné à combattre le SIDA.

## Revendications (Revendications pour l'(les) Etat(s) contractant(s) suivant(s): GR, ES)

1. Procédé pour la préparation de peptides de la protéine gag du VIH, caractérisé en ce que le peptides contiennent la séquence peptidique NPGLLETSEGCRQ et ne comportent pas plus de 50 aminoacides.

2. Procédé selon la revendication 1, caractérisé en ce que les peptides contiennent P4 ou P5.

3. Procédé selon la revendication 1 ou 2,. caractérisé en ce que la séquence peptidique NPGLLETSEGCRQ est raccourcie de jusqu'à 7 aminoacides à l'extrémité N-terminale et/ou à l'extrémité C-terminale, mais n'est pas plus courte que 6 aminoacides.

4. Procédé selon au moins l'une des revendications 1 à 3, caractérisé en ce que la séquence peptidique NPGLLETSEGCRQ comporte un ou plusieurs des remplacements d'aminoacides suivants: l'asparagine par la glutamine ou la glutamine par l'asparagine, la proline par l'hydroxyproline, la leucine par l'isoleucine ou la norleucine, l'acide glutamique par l'acide aspartique, la thréonine par la sérine ou la sérine par la thréonine, la cystéine par la sérine, l'arginine par la lysine, l'alanine par la glycine et/ou la méthionine par la norleucine.

5. Procédé pour la fabrication d'un médicament, caractérisé en ce que l'on utilise des peptides préparés selon au moins l'une des revendications 1 à 4.

6. Procédé pour la fabrication d'un médicament selon la revendication 5, caractérisé en ce que l'on utilise en outre un véhicule inerte.

7. Procédé pour la fabrication d'un médicament selon la revendication 5 ou 6, caractérisé en ce que le médicament est utilisé pour combattre le SIDA.
